(19) **Europäisches Patentamt**
**European Patent Office**
**Office européen des brevets**

(11) **EP 2 342 582 B1**

(12) **FASCICULE DE BREVET EUROPEEN**

(45) Date de publication et mention
de la délivrance du brevet:
**05.09.2012 Bulletin 2012/36**

(51) Int Cl.:
***G01S 7/52*** *(2006.01)*

(21) Numéro de dépôt: **09764270.6**

(86) Numéro de dépôt international:
**PCT/FR2009/052099**

(22) Date de dépôt: **30.10.2009**

(87) Numéro de publication internationale:
**WO 2010/049658 (06.05.2010 Gazette 2010/18)**

(54) **PROCEDE ET DISPOSITIF DE SONDAGE PAR PROPAGATION D'ONDES**

VERFAHREN UND VORRICHTUNG FÜR SONDIERUNG MITTELS WELLENAUSBREITUNG

METHOD AND DEVICE FOR PROBING BY WAVE PROPAGATION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **31.10.2008 FR 0857425**

(43) Date de publication de la demande:
**13.07.2011 Bulletin 2011/28**

(73) Titulaire: **Centre National de la Recherche Scientifique -
CNRS
75794 Paris Cedex 16 (FR)**

(72) Inventeurs:
• **FINK, Mathias**
**F-92190 Meudon (FR)**
• **MONTALDO, Gabriel**
**F-75009 Paris (FR)**
• **TANTER, Mickael**
**F-92220 Bagneux (FR)**

(74) Mandataire: **Cabinet Plasseraud
52, rue de la Victoire
75440 Paris Cedex 09 (FR)**

(56) Documents cités:
**US-A1- 2005 033 170     US-A1- 2006 241 429**

• **KARAMAN M ET AL: "Adaptive digital beamforming for phased array ultrasound imaging (medical US imaging)" 19911208; 19911208 - 19911211, 8 décembre 1991 (1991-12-08), pages 1207-1210, XP010094014**
• **MALLART R ET AL: "THE VAN CITTERT-ZERNIKE THEOREM IN PULSE ECHO MEASUREMENTS" THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 90, no. 5, 1 novembre 1991 (1991-11-01), pages 2718-2727, XP000236651 ISSN: 0001-4966 cité dans la demande**

EP 2 342 582 B1

Printed by Jouve, 75001 PARIS (FR)

**Description**

DOMAINE DE L'INVENTION

La présente invention est relative aux procédés et dispositifs de sondage par propagation d'ondes.

Plus particulièrement, l'invention concerne un procédé de sondage par propagation d'ondes, comprenant :

(a) une étape de mesure comportant plusieurs émissions (a1) au cours desquelles on fait émettre par un ensemble de transducteurs i (c'est-à-dire par tout ou partie des transducteurs de l'ensemble) des ondes incidentes dans un milieu diffusant, et plusieurs réceptions (a2) au cours desquelles on fait capter par l'ensemble de transducteurs (c'est-à-dire par tout ou partie des transducteurs de l'ensemble) des signaux représentatifs des ondes réverbérées par le milieu à partir de chaque onde incidente,
(b) une étape de traitement au cours de laquelle on traite les signaux captés.

On notera que les transducteurs susmentionnés peuvent être tout dispositif capable de transformer un signal électrique en une onde, quelle que soit la nature de l'onde.

L'étape de traitement susmentionnée permet par exemple de mesurer un paramètre caractéristique du milieu, et/ou de détecter un point singulier du milieu, et/ou de réaliser une image du milieu, et/ou déterminer des réponses impulsionnelles du milieu permettant ensuite de focaliser un signal en des endroits prédéterminés du milieu (par exemple à des fins de communication, de traitement thérapeutique ou autre).

ARRIERE-PLAN DE L'INVENTION

Les procédés de ce type sont utilisables notamment dans les systèmes de détection et d'imagerie, comme par exemple les sonars, les radars, les échographes, les systèmes d'imagerie optique adaptative, etc., aux fins d'imagerie médicale, de traitement thérapeutique, de contrôle non destructif des matériaux, en sismologie et imagerie du sous-sol, etc.

Ces procédés connus présentent de grandes difficultés de mise en oeuvre lorsque le milieu est aberrateur notamment du fait de son hétérogénéité, car dans ce cas il est difficile de focaliser efficacement les ondes en émission comme en réception.

Diverses méthodes ont déjà été proposées pour corriger les distorsions induites par les hétérogénéités du milieu imagé. Certaines sont fondées sur l'utilisation d'un point brillant (réflecteur) dans la zone de focalisation qui permet de corriger les distorsions - voir notamment Prada et al. ["The iterative time reversal process: A solution to self-focusing in the pulse echo mode", J. Acoust . Soc. Am. 90, 1119-1129, 1991, *X*P222986].

Dans le cas plus général, malheureusement on ne dispose pas de tel point brillant. A cet effet, des méthodes ont été développées par Mallart et al. ["The van Cittert-Zernike theorem in pulse echo measurements", J Acoustical Soc. Am., Vol 90, Issue 5, pp. 2718-2727, 1991, XP236651] pour exploiter la cohérence des ondes rétrodiffusées par le milieu, mais leur manque de robustesse a empêché leur implémentation industrielle.

RESUME DE L'INVENTION

La présente invention a notamment pour but de pallier ces inconvénients.

A cet effet, selon l'invention, au cours de l'étape de traitement, on considère plusieurs ensembles k de signaux réverbérés $B_k(i, t)$ provenant chacun sensiblement d'un point $r_k$ appartenant à une même zone d'isoplanétisme ZI $(r_0)$ qui est commune auxdits ensembles k de signaux et qui est elle-même relative à un point $r_0$ du milieu, les points $r_k$ étant soit différents les uns des autres quel que soit le milieu, soit confondus avec le point $r_0$ lorsque le milieu comporte des diffuseurs ayant un mouvement aléatoire, on recale temporellement les signaux réverbérés $B_k(i, t)$ des ensembles k si les points $r_k$ sont différents les uns des autres pour obtenir des signaux corrigés $B0_k(i, t)= B_k(i, t-G_i(r_k))$, les valeurs $G_i(r_k)$ étant des retards tels qu'en faisant émettre des signaux $e_k(i,t)=e_0(i,t+G_i(r_k))$ par les transducteurs i, l'onde incidente focaliserait sensiblement au point $r_k$, les signaux $e_0(i,t)$ étant des signaux de référence tels qu'en faisant émettre lesdits signaux de référence $e_0(i,t)$ par les transducteurs i, l'onde incidente focalise sensiblement au point $r_0$, on met en phase et on moyenne des signaux $B0_k(i,t)= B_k(i,t-G_i(r_k))$ (on notera que lorsque chaque point $r_k$ est confondu avec $r_0$, $G_{i(r_k)}=0$ et donc $B0_k(i,t )=B_k(i,t)$) pour obtenir des signaux moyens $Bf(i,t) = \sum_k A_k B0_k(i,t - c_k)$, où les

valeurs $c_k$ sont des retards permettant la mise en phase des signaux $B0_k$ et $A_k$ des coefficients de pondération (éventuellement, tous les coefficients $A_k$ peuvent être égaux à 1).

Grâce à ces dispositions, il est possible de supprimer les variations spatialement incohérentes du signal capté dues aux aberrations du milieu, de sorte que l'on peut ainsi s'affranchir des distorsions générées par les hétérogénéités du milieu, même dans les milieux très aberrateurs (par exemple la boîte crânienne en imagerie ultrasonore ou en traitement ultrasonore, ou encore les couches de graisse et de muscles dans le cas de l'imagerie ultrasonore ou du traitement ultrasonore de l'abdomen).

Le procédé selon l'invention est aisé à mettre en oeuvre, même dans les systèmes préexistants, et il s'avère particulièrement robuste.

On notera que les signaux réverbérés $B_k(i,t)$ ne sont pas forcément physiquement réverbérés et ne proviennent pas forcément physiquement des points $r_k$ : ces signaux peuvent en effet être :

- soit physiquement captés à partir d'ondes incidentes focalisées respectivement aux points $r_k$, auquel cas ils sont bien physiquement réverbérés et ils proviennent bien physiquement des points $r_k$,
- soit synthétisés à partir d'ondes incidentes non focalisées aux points $r_k$, auquel cas ces signaux synthétiques ne sont pas physiquement réverbérés à partir des points $r_k$ mais reproduisent ce que seraient des signaux physiquement réverbérés à partir des points $r_k$.

Dans divers modes de réalisation du procédé selon l'invention, on peut éventuellement avoir recours en outre à l'une et/ou à l'autre des dispositions suivantes :

- chaque ensemble k de signaux correspond à une émission k focalisée au point $r_k$, et au cours de chaque émission k, on émet des signaux $e_k(i,t)=e_0(i,t+G_i(r_k))$ ;
- les retards $G_i(r_k)$ sont tels qu'en faisant émettre des signaux $e_k(i,t)=e_0(i,t+G_i(r_k))$ par les transducteurs i, l'onde incidente focaliserait au point $r_k$ en supposant le milieu homogène ;
- les signaux de référence $e_0(i,t)$ sont déterminés comme si le milieu était homogène ;
- le retard $c_k$ est calculé par comparaison entre les signaux $B0_k(i,t)$ et le signal $e_0(i,t)$;
- le retard $c_k$ est calculé par comparaison entre les signaux $B0_k(i,t)$ ;
- les étapes de mesure et de traitement sont mises en oeuvre en plusieurs itérations successives j pour une même zone d'isoplanétisme $ZI(r_0)$ pour obtenir à chaque itération un signal moyen $Bf_j(i,t)$, le signal $e0(i,t)$ utilisé pour l'émission (a1) de chaque nouvelle itération j postérieure à l'itération initiale 1 étant déterminé à partir d'une estimation d'un retournement temporel $Bf_{j-1}(i,t)$ du signal moyen $Bf_{j-1}(i,t)$ déterminé à l'étape j-1 ;
- ladite estimation du retournement temporel est déterminée à partir d'un front d'onde du retournement temporel $Bf_{j-1}(i,-t)$ ;
- les signaux de référence $e_0(i,t)$ de l'itération initiale j = 1, sont déterminés comme si le milieu était homogène ;
- les points $r_k$ sont tous confondus avec le point $r_0$, les retards $G_i(r_k)$ étant égaux à 0, le milieu comportant des diffuseurs ayant un mouvement aléatoire ;
- les signaux réverbérés $B_k(i,t)$ sont construits par une méthode synthétique à partir des émissions et réceptions effectuées au cours de l'étape de mesure ;
- on détermine plusieurs signaux moyens $Bf(i,t)$ relatifs à différents points $r_0$ du milieu et on utilise lesdits signaux moyens $Bf(i,t)$ pour bâtir une image du milieu ;
- on utilise le signal moyen $Bf(i,t)$ pour calculer un paramètre propre au milieu ;
- ledit paramètre est la célérité de l'onde ;
- lesdites ondes sont choisies parmi les ondes ultrasonores, les ondes mécaniques (autres qu'ultrasonores) et les ondes électromagnétiques.

Par ailleurs, l'invention a également pour objet un dispositif pour la mise en oeuvre d'un procédé de sondage tel que défini ci-dessus, comprenant un ensemble de transducteurs i adaptés pour émettre une onde incidente dans un milieu diffusant et pour capter des signaux représentatifs d'une onde réfléchie réverbérée par le milieu à partir de l'onde incidente, le dispositif comportant des moyens de commande adaptés pour faire effectuer par ledit ensemble de transducteurs au moins une mesure comportant plusieurs émissions (a1) au cours desquelles on fait émettre par l'ensemble de transducteurs i des ondes incidentes dans le milieu, et plusieurs réceptions (a2) au cours desquelles on capte les signaux représentatifs des ondes réfléchies réverbérées par le milieu à partir de chaque onde incidente,

et le dispositif comportant en outre des moyens de traitement adaptés pour effectuer une étape de traitement (b) au cours de laquelle on considère plusieurs ensembles k de signaux réverbérés $B_k(i,t)$ provenant chacun sensiblement d'un point $r_k$ appartenant à une même zone d'isoplanétisme $ZI(r_0)$ qui est commune auxdits ensembles k de signaux et qui est elle-même relative à un point $r_0$ du milieu, les points $r_k$ étant soit différents les uns des autres quel que soit le milieu, soit confondus avec le point $r_0$ lorsque le milieu comporte des diffuseurs ayant un mouvement aléatoire, lesdits moyens de traitement étant adaptés pour :

- recaler temporellement les signaux réverbérés $B_k(i,t)$ des ensembles k si les points $r_k$ sont différents les uns des autres pour obtenir des signaux corrigés $B0_k(i, t)=B_k(i,t-G_i(r_k))$, les valeurs $G_i(r_k)$ étant des retards tels qu'en faisant émettre des signaux $e_k(i,t)=e_0(i,t+G_i(r_k))$ par les transducteurs i, l'onde incidente focaliserait sensiblement au point $r_k$, les signaux $e_0(i,t)$ étant des signaux de référence tels qu'en faisant émettre lesdits signaux de référence $e_0(i,t)$ par les transducteurs i, l'onde incidente focalise sensiblement au point $r_0$,

- et mettre en phase et moyenner des signaux $B0_k(i,t)=B_k(i,t-G_i(r_k))$ pour obtenir des signaux moyens

$$Bf(i,t) = \sum_k A_k B0_k(i, t - c_k) \, ,$$ où les valeurs $c_k$ sont des retards permettant la mise en phase des signaux $B0_k$ et $A_k$ des coefficients de pondération.

BREVE DESCRIPTION DES DESSINS

D'autres caractéristiques et avantages de l'invention apparaîtront au cours de la description suivante de plusieurs de ses modes de réalisation, donnés à titre d'exemples non limitatifs, en regard des dessins joints.

Sur les dessins :

- la figure 1 est une vue schématique d'un dispositif pour la mise en oeuvre d'un procédé selon un mode de réalisation de l'invention,
- la figure 2 est un schéma représentant les fronts d'onde successifs d'une onde acoustique incidente $e_1$ focalisée sur un point $X_1$ du milieu à étudier,
- les figures 3 et 4 sont des vues similaires à la figure 2, montrant les fronts d'onde successifs de deux composantes ($Bf_1$, $Bs_1$ pour la figure 3, $Bf_2$, $Bs_2$ pour la figure 4) de l'onde rétrodiffusée par le milieu, suite à deux émissions successives de la même onde incidente e1 focalisée au même point $X_1$,
- la figure 5 schématise le traitement par moyennage appliqué à un groupe de n signaux reçus par chaque transducteur i après un groupe de n tirs d'ondes focalisées au même point $X_1$,
- la figure 6 est une vue agrandie montrant différents points $r_k$ vers lesquels on peut focaliser successivement l'onde incidente dans une petite zone dite zone d'isoplanétisme autour d'un point central $X_1$ pour obtenir un groupe de signaux captés auquel peut être appliqué le traitement de moyennage schématisé sur la figure 5,
- et les figures 7 et 8 sont des exemples d'images du milieu de la figure 1, obtenues respectivement par échographie classique et en utilisant le procédé selon l'invention.

DESCRIPTION DETAILLEE

Sur les différentes figures, les mêmes références désignent des éléments identiques ou similaires.

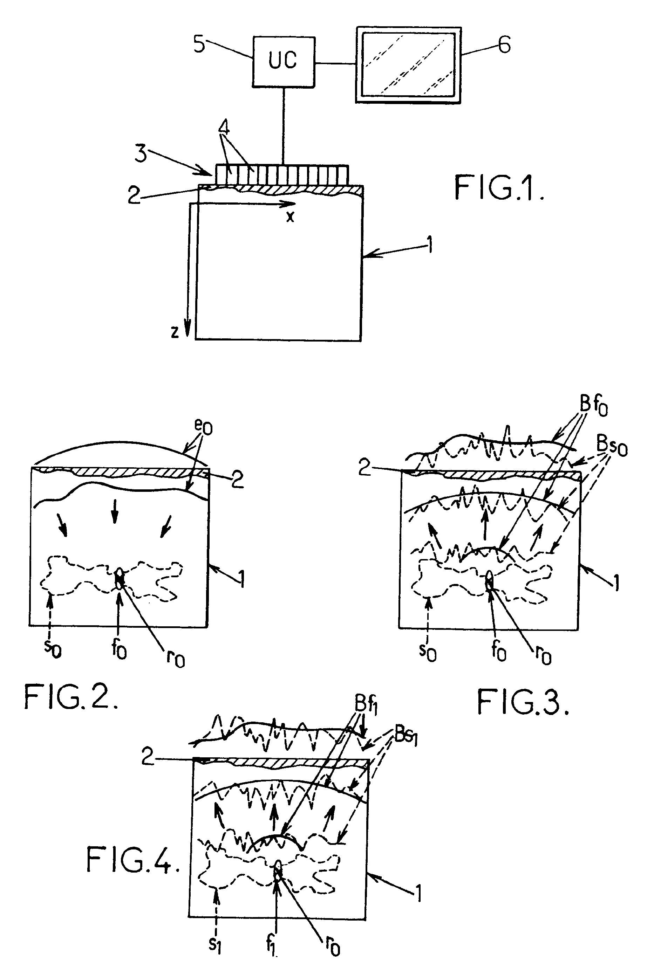

La figure 1 montre un exemple de dispositif adapté pour sonder un milieu 1 par émission et réception d'ondes. L'invention sera décrite plus particulièrement ci-après dans le cas où les ondes en question sont des ondes ultrasonores de compression (par exemple de fréquences comprises entre 2 et 4 MHz). On notera toutefois que l'invention est également applicable à des ondes de toute nature, par exemple des ondes mécaniques autres que des ondes ultrasonores de compression, des ondes électromagnétiques, ou autres.

Le milieu 1 est diffusant pour les ondes considérées, et plus particulièrement contient des diffuseurs 2 répartis de manière aléatoire et capables de réfléchir les ondes émises dans le milieu 1.

Le milieu 1 en question peut être par exemple une partie du corps humain, et les diffuseurs peuvent être notamment des particules de petite taille, non résolues, contenues dans le milieu 1 (en échographie de tels diffuseurs engendrent des images dites de «speckle»). Bien entendu, le milieu 1 à sonder pourrait être autre, par exemple une partie d'un objet industriel dont on souhaite contrôler la structure dans le cadre d'un contrôle non destructif.

Le milieu 1 comporte en outre, dans l'exemple considéré ici, une couche aberratrice 2. Un exemple de telle couche aberratrice est constitué par le crâne dans le cas de l'imagerie ultrasonore du cerveau ou des couches de graisse et de muscles dans le cas de l'imagerie ultrasonore de l'abdomen, mais plus généralement, il peut s'agir de n'importe quelle couche hétérogène par rapport au reste du milieu 1.

Le dispositif de sondage représenté sur la figure 1 comporte un ensemble 3 de transducteurs 4, par exemple un réseau linéaire de transducteurs piézoélectriques ultrasonores pouvant se présenter classiquement sous la forme d'une barrette rigide mise en contact avec le milieu 1 (et plus particulièrement avec la couche aberratrice 2 dans l'exemple considéré). Dans l'exemple représenté, cette barrette s'étend selon un axe x et permet de sonder le milieu 1 dans un plan (x,z), z étant un axe perpendiculaire à x et s'étendant dans la profondeur du milieu 1.

Le réseau 3 de transducteurs comporte un nombre N de transducteurs, N pouvant par exemple être compris entre 100 et 500. On peut par exemple utiliser une barrette d'une centaine de transducteurs 4 ayant chacun une largeur d'environ de largeur 0.39 mm. On notera que les transducteurs 4 sont ici des transducteurs ultrasonores capables de transformer un signal électrique en ondes ultrasonores de compression ou inversement, mais au sens du présent brevet, on appellera transducteur plus généralement tout dispositif capable de transformer un signal électrique en une onde de quelque nature que ce soit (onde mécanique, onde électromagnétique, onde optique, etc.) et inversement.

Chaque transducteur 4 du réseau 3 peut être commandé individuellement par une unité centrale 5 (UC) comprenant par exemple des moyens numériques de traitement de signaux, cette unité centrale 5 pouvant par exemple être adaptée pour présenter une image du milieu 1 sur un écran 6.

Pour sonder le milieu 1, l'unité centrale 5 envoie aux transducteurs 4 des signaux électriques qui sont transformés par lesdits transducteurs en ondes émises dans le milieu 1, en l'occurrence des ondes ultrasonores de compression, et ces ondes sont partiellement réfléchies par les diffuseurs contenus dans le milieu. Une partie des ondes diffusées (ou échos) revient ainsi vers les transducteurs 4 qui les captent et les transforment en signaux électriques de réception traités ensuite par l'unité centrale 5.

Le procédé selon l'invention prévoit de corriger les effets de la couche aberratrice 2, en émettant vers chaque point $r_0$ à étudier du milieu 1, un groupe de n ondes incidentes focalisées vers ce point ou à proximité, n étant un entier de préférence supérieur à 5 et par exemple compris entre 5 et 20, notamment entre 10 et 15.

La figure 2 schématise l'émission d'une première onde incidente $e_0(x,z,t)$ focalisée vers un point $r_0$ du milieu 1. Cette onde incidente est générée en faisant émettre par chaque transducteur i du réseau 3, un signal $e_0(i,t)$ qui est déterminé en appliquant à un signal de référence, une loi de retard cylindrique. Cette loi de retard est déterminée classiquement pour compenser les différences de marche entre chaque transducteur i et le point focal $r_0$, en supposant a priori qu'il n'y a pas d'aberration (à tort, et comme le fait tout échographe conventionnel).

La qualité de focalisation est toutefois fortement dégradée par les hétérogénéités introduites par la couche aberratrice 2, ce qui se traduit par une importante déformation du front d'onde après la traversée de la couche aberratrice 2, comme représenté sur la figure 2. En particulier, le niveau de lobes secondaires de la tache focale est important. Au point focal $r_0$, l'onde peut être décomposée en la somme de deux termes : le signal de focalisation $f_0(x,z,t)$ au point focal r0 (c'est-à-dire le signal qui serait obtenu en l'absence de couche aberratrice) et le signal $s_0(x,z,t)$ correspondant aux lobes secondaires (aux «imperfections» de la focalisation).

Comme représenté sur la figure 3, les signaux rétrodiffusés reçus sur le réseau de transducteurs peuvent s'exprimer sous la forme :

$$B_0(x,t) = Bf_0(x,z,t) + Bs_0(x,z,t)$$

où $Bf_0(x,t)$ correspond à la rétrodiffusion du signal de focalisation et $Bs_0(x,t)$ à la rétrodiffusion des lobes décrivant la dégradation de focalisation.

Selon le théorème de van Cittert-Zernike [voir en particulier : R. Mallart and M. Fink, "The van Cittert-Zernike theorem in pulse echo measurements", J. Acoustical Soc. Am. , Vol 90, Issue 5, pp. 2718-2727, 1991], la cohérence spatiale des signaux rétrodiffusés par un ensemble de diffuseurs répartis aléatoirement dans l'espace est inversement proportionnelle à la taille de la source, de sorte que le signal rétrodiffusé par la tache focale (équivalente à une petite source) est un signal cohérent, et les signaux rétrodiffusés par les lobes (constituant une source étendue) est un signal incohérent.

Si la position des diffuseurs contenus dans le milieu 1 change de manière aléatoire entre deux tirs d'ondes ultrasonore par le réseau 3 de transducteurs (par exemple si le milieu 1 est un milieu vivant ayant une certaine mobilité ou un milieu fluide en mouvement, par exemple du sang), une émission ultérieure d'une nouvelle onde incidente $e_1(x,z,t)$ de forme identique à la première onde incidente $e_0(x,z,t)$, se traduit par une nouvelle réalisation des signaux rétrodiffusés $B_1(x,t) = Bf_1(x,t) + Bs_1(x,t)$.

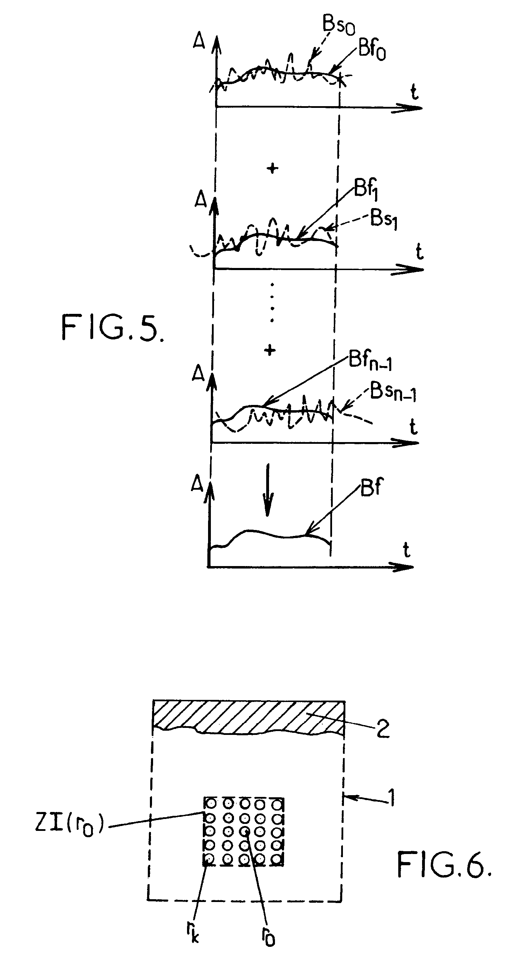

On peut ainsi effectuer un groupe d'émissions comprenant n tirs d'ondes incidentes $e_0(x,z,t)$, $e_1(x,z,t)$, ... $e_k(x,z,t)$, ... $e_{n-1}(x,z,t)$ focalisés vers le point cible r0, puis moyenner les différents signaux rétrodiffusés $B_0(x,z,t)$, $B_1(x,z,t)$, ... $B_k(x,z,t)$, ... $B_{n-1}(x,z,t)$ (formant un même groupe de réceptions correspondant au groupe susmentionné de n émissions), comme représenté sur la figure 5. Lors du calcul de cette moyenne, la part cohérente des signaux est ajoutée de manière constructive, mais la part incohérente est sommée de manière destructive et est finalement éliminée lors du calcul de la moyenne :

$$\frac{1}{n}\sum_k Bs_k(x,z,t) \to 0 \; : \; Bf = \frac{1}{n}\sum_k B_k(x,z,t) \cong \frac{1}{n}\sum_k Bf_k(x,z,t) \; .$$

On notera que la moyenne susmentionnée pourrait être une moyenne pondérée utilisant des coefficients de pondération $A_k$.

En pratique, on moyenne les signaux rétrodiffusés successifs $Bf_k(i,t)$ captés après chaque tir k, par chaque transducteur i du réseau 3. Le signal obtenu Bf(i,t) par moyennage pour chaque transducteur i, est représentatif du signal cohérent Bf susmentionné :

$$Bf(i,t) = \frac{1}{n}\sum_k B_k(i,t) \cong \frac{1}{n}\sum_k Bf_k(i,t) \; .$$

Ce processus peut être répété pour une pluralité de points cibles r0 dans le milieu 1. Pour des applications d'imagerie, on peut ainsi répéter le processus pour une matrice de points cibles r0 couvrant toute la zone à imager.

Les signaux cohérents Bf(i,t) calculés par moyennage pour chaque point r0 du milieu 1 peuvent être utilisés ensuite classiquement soit pour construire une image du milieu 1, soit pour pouvoir émettre ultérieurement une onde focalisée précisément en un point particulier du milieu (par exemple aux fins de traitement thérapeutique ou autre), ou encore pour calculer un paramètre du milieu (par exemple la célérité des ondes concernées, notamment les ondes ultrasonores de compression dans l'exemple particulier décrit ici).

Le procédé qui vient d'être décrit peut être encore perfectionné sur deux aspect :

- Moyennage de zone : dans la plupart des applications, le milieu ne change pas et il n'est pas possible d'avoir différentes réalisations temporelles du milieu aléatoire : on peut dans ce cas effectuer un moyennage par zone, en utilisant différentes réalisations spatiales du milieu aléatoire obtenues en focalisant successivement l'onde incidente des différentes émissions d'un même groupe d'émissions, en plusieurs points situés dans une petite zone dite d' «isoplanétisme» autour du point cible $r_0$ de ce groupe d'émissions ;
- Rephasage : notamment lorsque le milieu change, les ondes $f_k(x, z, t)$ et $f_k(x,z,t)$ d'un même groupe d'émission ne sont pas en phase : les signaux captés doivent donc alors préférentiellement être remis en phase avant moyennage.

**Moyennage de zone**

Dans un milieu statique, il est impossible d'obtenir différentes réalisations aléatoires des signaux rétrodiffusés dans un même groupe de réception (correspondant à un même point $r_0$ du milieu 1), en utilisant des ondes incidentes focalisées exactement au point ro.

Pour résoudre ce problème et pouvoir déterminer les signaux moyens Bf(i,t) susmentionnés pour chacun des points cibles $r_0$ à sonder, on focalise les ondes incidentes en différents points $r_k$ du milieu autour du point de focalisation cible concerné $r_0(x_0,z_0)$, dans une petite zone $ZI(r_0)$ entourant le point cible concerné $r_0(x_0,z_0)$.

Cette petite zone $ZI(r_0)$, dite zone d'isoplanétisme, est telle que l'onde incidente puisse être focalisée efficacement en $r_k$ par simple angulation (c'est-à-dire par application de retards sur les différents transducteurs 4) par rapport à l'onde incidente focalisée en $r_0$. Plus précisément, on peut définir la zone d'isoplanétisme $ZI(r_0)$ par le fait que les fronts d'onde rétrodiffusés émanant de tout point de cette zone sont identiques à environ un quart de longueur d'onde près, au décalage temporel près lié à l'écart entre les points considérés de la zone d'isoplanétisme.

On notera que dans le cas décrit ci-dessus où toutes les ondes incidentes d'un même groupe d'émissions sont focalisées exactement au même point $r_0$, toutes ces ondes incidentes sont a fortiori focalisées dans la zone d'isoplanétisme $ZI(r_0)$ attachée à ce point $r_0$.

Dans le cas considéré ici d'une focalisation en plusieurs points $r_k$, les n émissions du même groupe d'émissions sont donc focalisées respectivement sur $r_0$ et sur d'autres points $r_k$ de la zone d'isoplanétisme $ZI(r_0)$, soit au total n points (n peut être de préférence supérieur à 5 et par exemple compris entre 5 et 20, notamment entre 10 et 15, comme dans l'exemple précédemment décrit). Les signaux $e_k(i,t)$ émis par les transducteurs 1 du réseau 3 s'expriment sous la forme : $e_k(i,t)=e_0(i,t+G_i(r_k))$, où $G_i(r_k)$ est un retard appliqué au signal du transducteur i par rapport au signal de référence susmentionné $e_0(i,t)$ ($e_0(i,t)$ est le signal permettant de focaliser en $r_0$) pour que l'onde incidente focalise sensiblement en $r_k$. Ce retard $G_i(r_k)$ peut par exemple être déterminé comme si le milieu 1 était homogène.

Au cours des différents tirs d'ondes incidentes vers la zone d'isoplanétisme $ZI(r_0)$, lorsqu'on focalise au point

$r_0(x_0,z_0)$, le signal rétrodiffusé est $B_0(x,t)=Bf_0(x,t)+Bs_0(x,t)$ comme noté ci-dessus.

En revanche, lorsqu'on focalise l'onde incidente sur un point $r_k$ distinct de $r_0$ et appartenant à la zone d'isoplanétisme ZI(r0) [ce point ayant par exemple pour coordonnées $r_k(x_0+\Delta x,z_0)$ s'il est à même profondeur $z_0$ que $r_0$], on obtient une réalisation différente de la rétrodiffusion parce qu'on illumine une zone différente de diffuseurs aléatoirement placés dans la direction $x$ : $B_k (x, t) = Bf_k (x + \Delta x, t) + Bs_k (x + \Delta x, t)$.

A partir de ce groupe de n émissions, on obtient comme dans le cas précédemment décrit, un groupe de n réceptions correspondant à la zone d'isoplanétisme ZI($r_0$), ces n réceptions se traduisant par i*n signaux captés $B_k(i,t)$.

Ces signaux sont d'abord recalés temporellement entre eux pour corriger le déphasage introduit par le fait que les ondes incidentes sont focalisées en des points $r_k$ légèrement différents. A cet effet, on calcule des signaux corrigés $B0_k(i,t)=B_k(i,t-G_i(r_k))$. On notera que cette formule est exacte même dans le cas précédemment évoqués où on focalise toujours au même point $r_0$, puisque dans ce cas on a $r_k=r_0$, $G_i(r_k)=0$ et $B0_k(i,t)=B_k(i,t)$.

Enfin, on met en phase et on moyenne les signaux corrigés $B0_k(i,t)$ pour obtenir des signaux moyens

$$\mathrm{Bf}(i,t) = \sum_k A_k B0_k (i, t - c_k) \text{,}$$ où les valeurs $c_k$ sont des retards permettant la mise en phase des signaux $B0_k$

et $A_k$ des coefficients de pondération (éventuellement tous égaux à 1). Les retards $C_k$ permettant le rephasage sont déterminés comme explicité ci-après dans la partie « rephasage ».

Comme expliqué précédemment, ce processus peut être répété pour une pluralité de points cibles $r_0$ pour couvrir toute la zone à sonder du milieu 1.

## Rephasage

Entre deux signaux rétrodiffusés $B_k(x,z,t)$ et $B_{k'}(x,z,t)$ provenant de deux tirs différents d'un même groupe correspondant à deux réalisations différentes des diffuseurs (c'est-à-dire à deux états aléatoires différents des diffuseurs), les signaux cohérents $Bf_k(x,z,t)$ et $Bf_{k'}(x,z,t)$ sont pratiquement les mêmes, mais il peut exister une différence de phase entre les deux même après recalage temporel, par exemple du fait que le milieu a changé entre deux tirs ou simplement du fait que le recalage temporel ne permet pas de rephaser entièrement deux signaux rétrodiffusés après focalisation en deux points distincts compte tenu de l'hétérogénéité du milieu.

Il est alors utile de rephaser les signaux avant moyennage. Cette opération de rephasage peut être faite par différentes méthodes, notamment par des méthodes de comparaison entre signaux rétrodiffusés et par des méthodes de comparaison avec le signal de référence $e_0$.

S'agissant de la comparaison entre signaux, on peut calculer par exemple une corrélation temporelle entre $B0_k$ et $B0_{k'}$, par exemple entre $B0_k$ et $B0_0$ :

$$Ck(\tau) = \int B0_k (i,t) B0_0 (i,t-\tau) dt$$

$Ck(\tau)$ possède un maximum pour $\tau=c_k$, qui est la valeur du déphasage $c_k$ entre $B0_k(i,t)$ et $B0_0(i,t)$, déphasage qu'il faut appliquer à chaque signal corrigé $B0_k(i,t)$ pour rephaser tous les signaux $B0_k$ entre eux.

S'agissant de la comparaison avec le signal de référence $e_0$, on peut par exemple calculer une corrélation temporelle $C(\tau)$ entre la retournée temporelle $B0_k(i,-t)$ et $e_0(i,t)$ ($Ck(\tau) = \int B0_k (i,-t)e_0 (i, t-\tau) dt$), cette formule ayant un maximum pour $\tau=c_k$ qui est le retard à appliquer à chaque signal corrigé $B0_k(i,t)$ pour rephaser tous les signaux $B0_k(i,t)$ entre eux.

## Procédure itérative

Une fois obtenus les signaux cohérents $Bf(i,t)$ correspondant à chaque point cible $r_0$ du milieu 1, il est possible de construire une nouvelle émission qui focalise au point $r_0$ avec une focalisation meilleure que la focalisation initiale. Ce nouveau signal peut être réalisé en retournant temporellement le signal $Bf(i,t)$ : on prend alors comme signal de référence $e_0(i,t)=Bf(i,-t)$, puisque ce signal focalise en $r_0$.

On peut alors réitérer les étapes susmentionnées de mesure (émission / réception) et de traitement des signaux reçus en plusieurs itérations successives j pour chaque zone d'isoplanétisme ZI($r_0$) pour obtenir à chaque itération un signal moyen $Bf_j(i,t)$ (c'est-à-dire le signal moyen Bf mentionné précédemment, calculé pour l'itération j), le signal $e0(i,t)$ utilisé pour l'émission (a1) de chaque nouvelle itération j postérieure à l'itération initiale (j=1) étant déterminé à partir d'une estimation d'un retournement temporel $Bf_{j-1}(1,-t)$ du signal moyen $Bf_{j-1}(i,t)$ déterminé à l'étape j-1.

Au bout de quelques itérations, généralement 3 à 4, on converge vers une valeur stable des $Bf(i,t)$.

On notera qu'au cours de ce processus itératif, on détermine généralement une simple approximation du

retournement temporel Bf$_{j-1}$(i,-t) lors du passage de l'itération j-1 à l'itération j, ne serait-ce que parce que l'on travaille sur des échantillonnages des signaux.

Cette approximation peut éventuellement être simplement le front d'onde de la retournée temporelle Bf$_{j-1}$(i,-t). Ainsi, dans le cas où on dispose uniquement de transducteurs 4 de type pulseurs, on peut approximer *Bf(x,-t)* par une simple loi de retard et d'amplitude *Bf(x,-t)≈A(x)P(t-r(t))=e$_2$(x,t)* où *A(x)* est la loi d'amplitude, *r (t)* est la loi de retard et *P (*t) est la forme de l'impulsion identique sur chaque voie.

**Utilisation de signaux synthétiques**

Dans toutes les variantes susmentionnées, on peut éventuellement émettre des ondes incidentes non focalisées sur le ou les points r$_k$. Dans ce cas, il est possible de construire a posteriori des signaux réverbérés B$_k$(i,t) par des méthodes synthétiques classiques à partir des émissions et réceptions effectuées au cours de l'étape de mesure (par exemple par combinaison linéaire de signaux captés à partir des ondes incidentes non focalisées), comme si ces signaux B$_k$(i,t) avaient été captés suite à une émission d'onde focalisée en r$_k$. Ces signaux synthétisés B$_k$(i,t) sont ensuite traités comme explicité ci-dessus.

**Application à la correction d'aberrations en imagerie échographique**

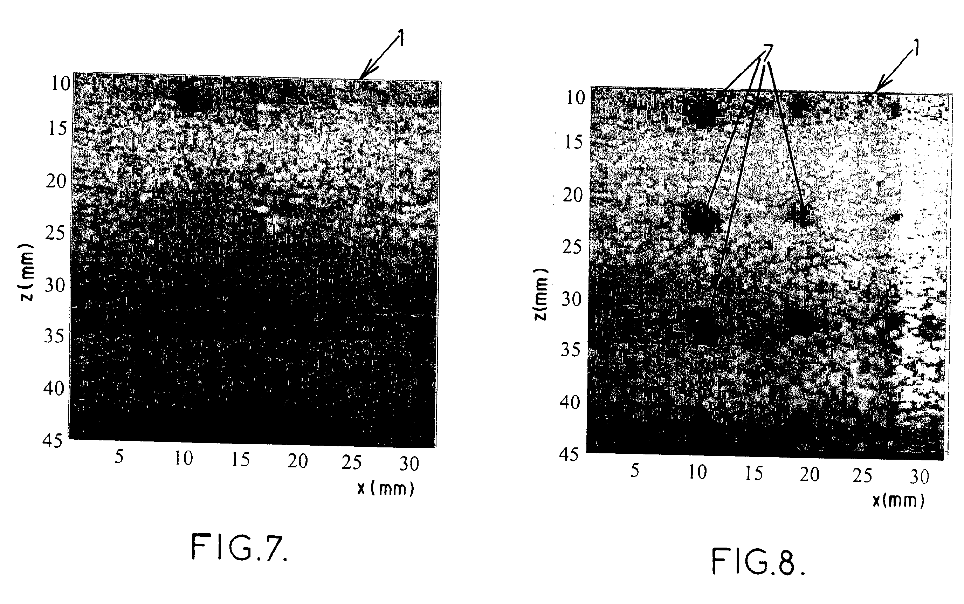

Les figures 7 et 8 montrent respectivement deux images échographiques d'un milieu 1 constitué par un bloc de gélatine contenant une multitude de petits diffuseurs (poudre d'Agar) beaucoup plus petits que la longueur d'onde. Un milieu aberrateur de 3 mm d'épaisseur de forme irrégulière est placé entre le réseau de transducteurs et le milieu. Il génère des variation de phase importante sur les faisceaux ultrasonores (> 2$\pi$).

Sur la figure 7, l'image est obtenue par les techniques classiques d'échographie (formation de voies) et ne permet de distinguer aucun détail. Sur la figure 7, l'image est obtenue en mettant en oeuvre le précédé selon l'invention de façon itérative, après 3 à 4 itérations : les signaux cohérents obtenus par moyennage sont représentatifs des fonctions de Green du milieu et permettent d'obtenir une image de qualité fortement supérieure. On peut ainsi voir clairement sur l'image corrigée les zones anéchoïques 7 qui étaient invisibles sur l'image standard.

**Application au calcul de la vitesse moyenne de propagation de l'onde en milieu homogène ou stratifié**

Considérons un milieu homogène de vitesse de propagation d'onde inconnue. On peut calculer cette vitesse en utilisant la contribution cohérente des ondes rétrodiffusées obtenue à partir de notre technique innovante. Dans un milieu homogène, la partie cohérente du signal rétrodiffusé venant du point r$_0$ correspond à :

$$Bf\,(x,t) = \frac{1}{r}P(t - r/c) \qquad \text{avec} \qquad r^2 = (x - x_0)^2 + z_0^2$$

La célérité de l'onde *c* peut être déduite en faisant correspondre les signaux captés moyennés Bf(i,t) avec une courbe analytique provenant d'un modèle de propagation. Cette application peut être aisément étendue au cas des milieux hétérogènes stratifiés.

**Revendications**

1. Procédé de sondage par propagation d'ondes, comprenant :

(a) une étape de mesure comportant plusieurs émissions (a1) au cours desquelles on fait émettre par un ensemble (3) de transducteurs i des ondes incidentes dans un milieu diffusant (1), et plusieurs réceptions (a2) au cours desquelles on fait capter par l'ensemble (3) de transducteurs des signaux représentatifs des ondes réverbérées par le milieu (1) à partir de chaque onde incidente,
(b) une étape de traitement au cours de laquelle on traite les signaux captés,

**caractérisé en ce qu'**au cours de l'étape de traitement, on considère plusieurs ensembles k de signaux réverbérés B$_k$(i,t) provenant chacun sensiblement d'un point r$_k$ appartenant à une même zone d'isoplanétisme ZI(r$_0$) qui est commune auxdits ensembles k de signaux et qui est elle-même relative à un point r$_0$ du milieu, les points r$_k$ étant

soit différents les uns des autres quel que soit le milieu, soit confondus avec le point $r_0$ lorsque le milieu comporte des diffuseurs ayant un mouvement aléatoire,

on recale temporellement les signaux réverbérés $B_k(i,t)$ des ensembles k si les points $r_k$ sont différents les uns des autres pour obtenir des signaux corrigés $B0_k(i,t)=B_k(i,t-G_i(r_k))$, les valeurs $G_i(r_k)$ étant des retards tels qu'en faisant émettre des signaux $e_k(i,t)=e_0(i,t+G_i(r_k))$ par les transducteurs i, l'onde incidente focaliserait sensiblement au point $r_k$, les signaux $e_0(i,t)$ étant des signaux de référence tels qu'en faisant émettre lesdits signaux de référence $e_0(i,t)$ par les transducteurs i, l'onde incidente focalise sensiblement au point $r_0$,

on met en phase et on moyenne des signaux $B0_k(i, t)= B_k(i,t-G_i(r_k))$ pour obtenir des signaux moyens $Bf(i,t) = \sum_k A_k B0_k(i, t - c_k)$, où les valeurs $c_k$ sont des retards permettant la mise en phase des signaux $B0_k$ et $A_k$ des coefficients de pondération.

**2.** Procédé selon la revendication 1, dans lequel chaque ensemble k de signaux correspond à une émission k focalisée au point $r_k$, et au cours de chaque émission k, on émet des signaux $e_k(i,t)=e_0(1,t+G_i(r_k))$.

**3.** Procédé selon la revendication 1, dans lequel les retards $G_i(r_k)$ sont tels qu'en faisant émettre des signaux $e_k(i,t)=e_0(i,t+G_i(r_k))$ par les transducteurs i, l'onde incidente focaliserait au point $r_k$ en supposant le milieu homogène.

**4.** Procédé selon la revendication 1, dans lequel les signaux de référence $e_0(i,t)$ sont déterminés comme si le milieu était homogène.

**5.** Procédé selon la revendication 1, dans lequel le retard $c_k$ est calculé par comparaison entre les signaux $B0_k(i,t)$ et le signal $e_0(i,t)$.

**6.** Procédé selon la revendication 1 dans laquelle le retard $c_k$ est calculé par comparaison entre les signaux $B0_k(i,t)$.

**7.** Procédé selon la revendication 2, dans lequel les étapes de mesure et de traitement sont mises en oeuvre en plusieurs itérations successives j pour une même zone d'isoplanétisme $ZI(r_0)$ pour obtenir à chaque itération un signal moyen $Bf_j(i,t)$, le signal $e0(i,t)$ utilisé pour l'émission (a1) de chaque nouvelle itération j postérieure à l'itération initiale 1 étant déterminé à partir d'une estimation d'un retournement temporel $Bf_{j-i}(i,-t)$ du signal moyen $Bf_{j-1}(i,t)$ déterminé à l'étape j-1.

**8.** Procédé selon la revendication 7, dans lequel ladite estimation du retournement temporel est déterminée à partir d'un front d'onde du retournement temporel $Bf_{j-1}(i,t)$.

**9.** Procédé selon la revendication 7, dans lequel les signaux de référence $e_0(i,t)$ de l'itération initiale j=1, sont déterminés comme si le milieu était homogène.

**10.** Procédé selon le revendication 1, dans lequel les points $r_k$ sont tous confondus avec le point $r_0$, les retards $G_i(r_k)$ étant égaux à 0, le milieu comportant des diffuseurs ayant un mouvement aléatoire.

**11.** Procédé selon la revendication 1, dans lequel les signaux réverbérés $B_k(i,t)$ sont construits par une méthode synthétique à partir des émissions et réceptions effectuées au cours de l'étape de mesure.

**12.** Procédé selon la revendication 1, dans lequel on détermine plusieurs signaux moyens $Bf(i,t)$ relatifs à différents points $r_0$ du milieu et on utilise lesdits signaux moyens $Bf(i,t)$ pour bâtir une image du milieu.

**13.** Procédé selon la revendication 1, dans lequel on utilise le signal moyen $Bf(i,t)$ pour calculer un paramètre propre au milieu.

**14.** Procédé selon la revendication 13, dans lequel ledit paramètre est la célérité de l'onde.

**15.** Procédé selon la revendication 1, dans lequel lesdites ondes sont choisies parmi les ondes ultrasonores, les ondes mécaniques et les ondes électromagnétiques.

**16.** Dispositif pour la mise en oeuvre d'un procédé de sondage selon l'une quelconque des revendications précédentes, comprenant un ensemble (3) de transducteurs i adaptés pour émettre une onde incidente dans un milieu diffusant (1) et pour capter des signaux représentatifs d'une onde réfléchie réverbérée par le milieu (1) à partir de l'onde incidente, le dispositif comportant des moyens de commande (5) adaptés pour faire effectuer par ledit ensemble (3) de transducteurs au moins une mesure comportant plusieurs émissions (a1) au cours desquelles on fait émettre par l'ensemble (3) de transducteurs i des ondes incidentes dans le milieu (1), et plusieurs réceptions (a2) au cours desquelles on capte les signaux représentatifs des ondes réfléchies réverbérées par le milieu à partir de chaque onde incidente,

et le dispositif comportant en outre des moyens de traitement (5) adaptés pour effectuer une étape de traitement (b) au cours de laquelle on considère plusieurs ensembles k de signaux réverbérés $B_k(i,t)$ provenant chacun sensiblement d'un point $r_k$ appartenant à une même zone d'isoplanétisme $ZI(r_0)$ qui est commune auxdits ensembles k de signaux et qui est elle-même relative à un point $r_0$ du milieu, les points $r_k$ étant soit différents les uns des autres quel que soit le milieu, soit confondus avec le point $r_0$ lorsque le milieu comporte des diffuseurs ayant un mouvement aléatoire,

lesdits moyens de traitement (5) étant adaptés pour :

- recaler temporellement les signaux réverbérés $B_k(i,t)$ des ensembles k si les points $r_k$ sont différents les uns des autres pour obtenir des signaux corrigés $B0_k(i,t)= B_k(i,t-G_i(r_k))$, les valeurs $G_i(r_k)$ étant des retards tels qu'en faisant émettre des signaux $e_k(i,t)=e_0(i,t+G_i(r_k))$ par les transducteurs i, l'onde incidente focaliserait sensiblement au point $r_k$, les signaux $e_0(i,t)$ étant des signaux de référence tels qu'en faisant émettre lesdits signaux de référence $e_0(i,t)$ par les transducteurs i, l'onde incidente focalise sensiblement au point $r_0$,

- et mettre en phase et moyenner des signaux $B0_k(i,t)= B_k(1,t-G_i(r_k))$ pour obtenir des signaux moyens

$$Bf(i,t) = \sum_k A_k B0_k(i,t-c_k)\,,$$ où les valeurs $c_k$ sont des retards permettant la mise en phase des signaux $B0_k$ et $A_k$ des coefficients de pondération.

**Claims**

1. A method of exploring by wave propagation, which method comprises:

   (a) a measurement step including a plurality of emissions (a1) during which a set of transducers i are caused to emit incident waves into a diffusing medium (1), and a plurality of receptions (a2) during which the set of transducers are caused to pick up signals representative of the waves reverberated by the medium (1) from each incident wave; and
   (b) a processing step during which the picked-up signals are processed;

   **characterized in that**, during the processing step, consideration is given to a plurality of sets k of reverberated signals $B_k(i,t)$, each of which comes substantially from a point $r_k$ belonging to the same isoplanatic zone $ZI(r_0)$ that is common to said sets k of signals and that itself relatives to a point $r_0$ of the medium, the points $r_k$ being either different from one another regardless of the medium, or coinciding with the point $r_0$ when the medium includes diffusers having random motion;

   the reverberated signals $B_k(i,t)$ of the sets k are re-positioned in time if the points $r_k$ are different from one another in order to obtain corrected signals $B0_k(i,t)= B_k(i,t-G_i(r_k))$, where the values $G_i(r_k)$ are delays such that, by causing the transducers i to emit signals $e_k(i,t)=e_0(i,t+G_i(r_k))$, the incident wave focuses substantially at the point $r_k$, the signals $e_0(i,t)$ being reference signals such that, by causing the transducers i to emit said reference signals $e_0(i,t)$, the incident wave focuses substantially at the point $r_0$;

   the signals $B0_k(i,t)= B_k(i,t-G_i(r_k))$ are put into phase and averaged so as to obtain average signals

   $$Bf(i,t) = \sum_k A_k B0_k(i,t-c_k)$$

   where the values $c_k$ are delays making it possible to put the signals $B0_k$ into phase, and the values $A_k$ are weighting coefficients.

2. A method according to claim 1, in which each set k of signals corresponds to an emission k focused at the point $r_k$,

and, during each emission $\underline{k}$, signals $e_k(i,t)=e_0(i,t+G_i(r_k))$ are emitted.

3. A method according to claim 1, in which the delays $G_i(r_k)$ are such that, by causing signals $e_k(i,t)=e_0(i,t+G_i(r_k))$ to be emitted by the transducers $\underline{i}$, the incident wave focuses at the point $r_k$, assuming that the medium is homogeneous.

4. A method according to claim 1, in which the reference signals $e_0(i,t)$ are determined as if the medium were homogeneous.

5. A method according to claim 1, in which the delay $c_k$ is computed by comparison between the signals $B0_k(i,t$ and the signal $e_0(i,t)$.

6. A method according to claim 1, in which the delay $c_k$ is computed by comparison between the signals $B0_k(i,t)$.

7. A method according to claim 2, in which the measurement and processing steps are implemented in a plurality of successive iterations j for the same isoplanatic zone $ZI(r_0)$ so as to obtain, at each iteration, an average signal $Bf_j(i,t)$, the signal $e_0(i,t)$ used for the emission (a1) of each new iteration $\underline{j}$ subsequent to the initial iteration 1 being determined on the basis of an estimation of a time reversal $Bf_{j-i}(i,-t)$ of the average signal $Bf_{j-1}(i,t)$ determined in the step j-1.

8. A method according to claim 7, in which said estimation of the time reversal is determined on the basis of a wavefront of the time reversal $Bf_{j-1}(i,-t)$.

9. A method according to claim 7, in which the reference signals $e_0(i,t)$ of the initial iteration j=1, are determined as if the medium were homogeneous.

10. A method according to claim 1, in which the points $r_k$ all coincide with the point $r_0$, the delays $G_i(r_k)$ being equal to 0, and the medium including diffusers having random motion.

11. A method according to claim 1, in which the reverberated signals $B_k(i,t)$ are constructed by a synthesis method on the basis of the emissions and receptions made during the measurement step.

12. A method according to claim 1, in which a plurality of average signals $Bf(i,t)$ are determined that relate to different points $r_0$ of the medium, and said mean signals $Bf(i,t)$ are used to build an image of the medium.

13. A method according to claim 1, in which the average signal $Bf(i,t)$ is used to compute a parameter that is specific to the medium.

14. A method according to claim 13, in which said parameter is the wave propagation speed.

15. A method according to claim 1, in which said waves are selected from among ultrasonic waves, mechanical waves, and electromagnetic waves.

16. Apparatus for implementing a method of exploring according to any preceding claim, said apparatus comprising a set (3) of transducers i suitable for emitting an incident wave into a diffusing medium (1), and for picking up signals representative of a reflected wave reverberated by the medium from the incident wave, the apparatus further comprising control means (5) suitable for causing said set (3) of transducers to take at least one measurement including a plurality of emissions (a1) during which the set (3) of transducers i are caused to emit incident waves into the medium (1), and a plurality of receptions (a2) during which the signals representative of the reflected waves reverberated by the medium from each incident wave are picked up; and

the device further comprising processing means (5) adapted to performing a processing step (b) during which consideration is given to a plurality of sets k of reverberated signals $B_k(i,t)$, each of which comes substantially from a point $r_k$ belonging to the same isoplanatic zone $ZI(r_0)$ that is common to said sets $\underline{k}$ of signals and that itself relates to a point $r_0$ of the medium, the points $r_k$ being either different from one another regardless of the medium, or coinciding with the point $r_0$ when the medium includes diffusers having random motion;

said processing means (5) being adapted to:

· re-position in time the reverberated signals $B_k(i,t)$ of the sets $\underline{k}$ if the points $r_k$ are different from one another in order to obtain corrected signals $B0_k(i,t)= B_k(i,t-G_i(r_k))$, where the values $G_i(r_k)$ are delays such that, by causing the transducers i to emit signals $e_k(i,t)=e_0(i,t+G_i(r_k))$, the incident wave focuses substantially at the point $r_k$, the signals $e_0(i,t)$ being reference signals such that, by causing the transducers i to emit said reference signals $e_0(i,t)$, the incident wave focuses substantially at the point $r_0$; and to

· put into phase and average the signals $B0_k(i,t)= B_k(i,t-G_i(r_k))$ so as to obtain average signals

$$Bf(i,t) = \sum_k A_k B0_k(i, t - c_k)$$

where the values $c_k$ are delays making it possible to put the signals $B0_k$ into phase, and the values $A_k$ are weighting coefficients.

## Patentansprüche

1. Verfahren zur Sondierung durch Wellenausbreitung, umfassend:

   (a) einen Messschritt, der mehrere Emissionsvorgänge (a1) umfasst, bei denen eine Anordnung (3) von Wandlern i dazu gebracht wird, Wellen zu emittieren, die in ein diffusives Medium (1) einfallen, und mehrere Empfangsvorgänge (a2) umfasst, bei denen die Anordnung (3) von Wandlern dazu gebracht wird, Signale zu erfassen, die repräsentativ für Wellen sind, die, ausgehend von jeder einfallenden Welle, durch das Medium (1) reflektiert werden,
   (b) einen Verarbeitungsschritt, bei welchem die erfassten Signale verarbeitet werden,

   **dadurch gekennzeichnet, dass** bei dem Verarbeitungsschritt mehrere Sätze k von reflektierten Signalen $B_k(i, t)$ betrachtet werden, die alle im Wesentlichen von einem Punkt $r_k$ kommen, der zu dem selben isoplanaren Bereich $ZI(r_0)$ gehört, der den Sätzen k von Signalen gemeinsam ist, und der selbst auf einen Punkt $r_0$ des Mediums bezogen ist, wobei die Punkte $r_k$ entweder voneinander verschieden sind, unabhängig von dem Medium, oder mit dem Punkt $r_0$ zusammenfallen, wenn das Medium Streuzentren umfasst, die eine zufällige Bewegung aufweisen,
   die reflektierten Signale $B_k(i,t)$ der Sätze k zeitlich korrigiert werden, wenn die Punkte $r_k$ voneinander verschieden sind, um korrigierte Signale $B0_k(i,t)=B_k(i,t-G_i(r_k))$ zu erhalten, wobei die Werte $G_i(r_k)$ Verzögerungen sind, so dass, wenn die Wandler i dazu gebracht werden, Signale $e_k(i,t)=e_0(i,t+G_i(r_k))$ zu emittieren, die einfallende Welle ihren Brennpunkt im Wesentlichen im Punkt $r_k$ hat, wobei die Signale $e_0(i,t)$ Referenzsignale sind, so dass, wenn die Wandler i dazu gebracht werden, die Referenzsignale $e_0(i,t)$ zu emittieren, die einfallende Welle ihren Brennpunkt im Wesentlichen im Punkt $r_0$ hat,
   die Signale $B0_k(i,t) = B_k(i,t-G_i(r_k))$ in Phase gebracht und gemittelt werden, um gemittelte Signale $Bf(i,t)=\Sigma_k A_k B0_k(i,t-c_k)$ zu erhalten, wobei die Werte $c_k$ Verzögerungen sind, welche es ermöglichen, die Signale $B0_k$ in Phase zu bringen, und $A_k$ Gewichtungskoeffizienten sind.

2. Verfahren nach Anspruch 1, wobei jeder Satz k von Signalen einer Emission k entspricht, die im Punkt $r_k$ ihren Brennpunkt hat, und bei jeder Emission k Signale $e_k(i,t)=e_0(i,t+G_i(r_k))$ emittiert werden.

3. Verfahren nach Anspruch 1, wobei die Verzögerungen $G_i(r_k)$ derart sind, dass, wenn die Wandler i dazu gebracht werden, Signale $e_k(i,t)=e_0(i, t+G_i(r_k))$ zu emittieren, die einfallende Welle im Punkt $r_k$ ihren Brennpunkt hat, wenn man ein homogenes Medium voraussetzt.

4. Verfahren nach Anspruch 1, wobei die Referenzsignale $e_0(i,t)$ so bestimmt werden, als wäre das Medium homogen.

5. Verfahren nach Anspruch 1, wobei die Verzögerung $c_k$ durch Vergleich zwischen den Signalen $B0_k(i,t)$ und dem Signal $e_0(i,t)$ berechnet wird.

6. Verfahren nach Anspruch 1, wobei die Verzögerung $c_k$ durch Vergleich zwischen den Signalen $B0_k(i,t)$ berechnet wird.

7. Verfahren nach Anspruch 2, wobei der Messschritt und der Verarbeitungsschritt in mehreren aufeinander folgenden Iterationen j für einen gleichen isoplanaren Bereich $ZI(r_0)$ durchgeführt werden, um bei jeder Iteration ein gemitteltes Signal $Bf_j(i,t)$ zu erhalten, wobei das Signal $e_0(i,t)$, welches für die Emission (a1) von jeder neuen Iteration j nach einer Anfangsiteration 1 verwendet wird, ausgehend von einer Abschätzung einer zeitlichen Umkehr $Bf_{j-1}(i,-t)$ des im Schritt j-1 bestimmten gemittelten Signals $Bf_{j-1}(i, t)$ bestimmt wird.

8. Verfahren nach Anspruch 7, wobei die Abschätzung der zeitlichen Umkehr ausgehend von einer Wellenfront der zeitlichen Umkehr $Bf_{j-1}(i,-t)$ bestimmt wird.

9. Verfahren nach Anspruch 7, wobei die Referenzsignale $e_0(i,t)$ der Anfangsiteration j = 1 so bestimmt werden, als wäre das Medium homogen.

10. Verfahren nach Anspruch 1, wobei die Punkte $r_k$ alle mit dem Punkt $r_0$ zusammenfallen, die Verzögerungen $G_i(r_k)$ gleich Null sind, das Medium Streuzentren umfasst, die eine zufällige Bewegung aufweisen.

11. Verfahren nach Anspruch 1, wobei die reflektierten Signale $B_k(i, t)$ durch ein Synthese-Verfahren konstruiert werden, ausgehend von Emissionsvorgängen und Empfangsvorgängen, die während des Messschritts durchgeführt werden.

12. Verfahren nach Anspruch 1, wobei mehrere gemittelte Signale $Bf(i, t)$ relativ zu verschiedenen Punkten $r_0$ des Mediums bestimmt werden, und die gemittelten Signale $Bf(i, t)$ verwendet werden, um ein Bild des Mediums aufzubauen.

13. Verfahren nach Anspruch 1, wobei das gemittelte Signal $Bf(i, t)$ verwendet wird, um einen Parameter zu berechnen, der zu dem Medium gehört.

14. Verfahren nach Anspruch 13, wobei der Parameter die Fortpflanzungsgeschwindigkeit der Welle ist.

15. Verfahren nach Anspruch 1, wobei die Wellen ausgewählt sind aus den Ultraschallwellen, den mechanischen Wellen und den elektromagnetischen Wellen.

16. Vorrichtung für die Durchführung eines Verfahrens zur Sondierung nach einem der vorhergehenden Ansprüche, umfassend eine Anordnung (3) von Wandlern i, die angepasst sind, um eine einfallende Welle in ein diffusives Medium (1) zu emittieren, und um Signale zu empfangen, die repräsentativ für eine reflektierte Welle sind, die von dem Medium (1) ausgehend von der einfallenden Welle reflektiert wurde, wobei die Vorrichtung Steuer-/Regelmittel (5) umfasst, die angepasst sind, um durch die Anordnung (3) von Wandlern wenigstens eine Messung durchführen zu lassen, die mehrere Emissionsvorgänge (a1) umfasst, bei denen die Anordnung (3) von Wandlern i dazu gebracht wird, in das Medium (1) einfallende Wellen zu emittieren, und mehrere Empfangsvorgänge (a2) umfasst, bei denen die Signale erfasst werden, die repräsentativ für reflektierte Wellen sind, die durch das Medium ausgehend von jeder einfallenden Welle reflektiert worden sind,
und die Vorrichtung weiterhin Verarbeitungsmittel (5) umfasst, die angepasst sind, um einen Verarbeitungsschritt (b) durchzuführen, bei dem mehrere Sätze k von reflektierten Signalen $B_k(i,t)$ betrachtet werden, die jeweils im Wesentlichen von einem Punkt $r_k$ kommen, der zum selben isoplanaren Bereich $ZI(r_0)$ gehört, der den Sätzen k von Signalen gemeinsam ist und der selbst auf einen Punkt $r_0$ des Mediums bezogen ist, wobei die Punkte $r_k$ sich entweder voneinander unterscheiden, unabhängig vom Medium, oder mit dem Punkt $r_0$ zusammenfallen, wenn das Medium Streuzentren umfasst, die eine zufällige Bewegung aufweisen,
wobei die Verarbeitungsmittel (5) angepasst sind um:

- die reflektierten Signale $B_k(i,t)$ der Sätze k zeitlich zu korrigieren, wenn die Punkte $r_k$ voneinander unterschiedlich sind, um korrigierte Signale $B0_k(i,t)=B_k(i,t-G_i(r_k))$ zu erhalten, wobei die Werte $G_i(r_k)$ Verzögerungen sind, so dass, wenn die Wandler i dazu gebracht werden, Signale $e_k(i,t)=e_0(i,t+G_i(r_k))$ zu emittieren, die einfallende Welle ihren Brennpunkt im Wesentlichen im Punkt $r_k$ hat, wobei die Signale $e_0(i,t)$ Referenzsignale sind, so dass, wenn die Wandler i dazu gebracht werden, die Referenzsignale $e_0(i,t)$ zu emittieren, die einfallende Welle ihren Brennpunkt im Wesentlichen im Punkt $r_0$ hat, und die Signale $B0_k(i,t) =B_k(i,t-G_i(r_k))$ in Phase zu bringen und zu mitteln, um gemittelte Signale $Bf(i,t)=\Sigma_k A_k B0_k(i,t-c_k)$ zu erhalten, wobei die Werte $c_k$ Verzögerungen sind, welche es ermöglichen, die Signale $B0_k$ in Phase zu bringen, und $A_k$ Gewichtungskoeffizienten sind.

FIG.1.

FIG.2.

FIG.3.

FIG.4.

FIG.5.

FIG.6.

FIG.7.

FIG.8.

**RÉFÉRENCES CITÉES DANS LA DESCRIPTION**

**Littérature non-brevet citée dans la description**

- **Prada et al.** The iterative time reversal process: A solution to self-focusing in the pulse echo mode. *J. Acoust . Soc. Am.,* 1991, vol. 90, 1119-1129
- **Mallart et al.** The van Cittert-Zernike theorem in pulse echo measurements. *J Acoustical Soc. Am.,* 1991, vol. 90 (5), 2718-2727

- **R. Mallart ; M. Fink.** The van Cittert-Zernike theorem in pulse echo measurements. *J. Acoustical Soc. Am.,* 1991, vol. 90 (5), 2718-2727